(19) Europäisches Patentamt
European Patent Office
Office européen des brevets

(11) Publication number: **0 164 700**
**B1**

(12) **EUROPEAN PATENT SPECIFICATION**

(45) Date of publication of patent specification: **11.01.89**

(21) Application number: **85107042.5**

(22) Date of filing: **07.06.85**

(51) Int. Cl.$^4$: **C 07 C 93/14,**
C 07 C 103/178,
C 07 C 103/34,
A 61 K 31/135, A 61 K 31/16,
A 61 K 31/22

(54) **Bis(beta-hydroxy phenethyl)amines.**

(30) Priority: **15.06.84 GB 8415377**

(43) Date of publication of application:
**18.12.85 Bulletin 85/51**

(45) Publication of the grant of the patent:
**11.01.89 Bulletin 89/02**

(84) Designated Contracting States:
**BE CH DE FR GB IT LI NL SE**

(56) References cited:
**EP-A-0 023 385**
**EP-A-0 070 133**
**EP-A-0 099 707**
**EP-A-0 101 069**

(73) Proprietor: **BEECHAM GROUP PLC**
**Beecham House Great West Road**
**Brentford Middlesex TW8 9BD (GB)**

(72) Inventor: **Hindley, Richard Mark**
**19 Cockshot Road**
**Reigate Surrey, RH2 7HD (GB)**

(74) Representative: **Dayneswood, Trevor et al**
**Beecham Pharmaceuticals Patent and Trade Mark Department Biosciences Research Centre**
**Great Burgh Yew Tree Bottom Road**
**Epsom Surrey KT18 5XQ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to tertiary amines which have β-agonist activity, to processes for their production and to their use in medicine and agriculture.

Background art relevant to the present invention includes: European Patent Applications, Publication Numbers 0,023,385 and 0,070,133, which disclose certain secondary ethanolamine derivatives having anti-obesity and anti-hyperglycaemic activity. Also European Patent Application, Publication Number 0,101,069 discloses certain tertiary ethanolamine derivatives having anti-obesity and anti-hyperglycaemic activity.

According to the present invention there is provided a compound of formula (I):

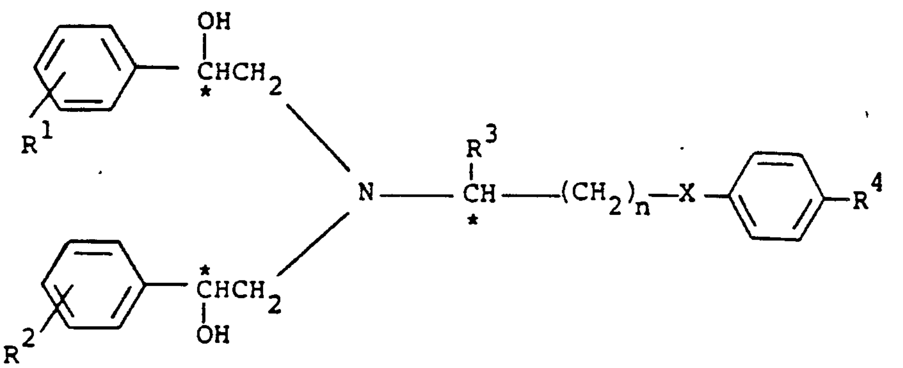

(I)

or a pharmaceutically acceptable salt thereof, in which

each of $R^1$ and $R^2$, which may be the same or different, is hydrogen, halogen, or trifluoromethyl,

$R^3$ is hydrogen or methyl,

$R^4$ is $-O(CH_2)_aCO_2H$, $-O(CH_2)_bOH$ or

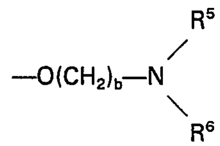

or an ester or amide derivative thereof, in which

a is an integer from 1 to 6

b is an integer from 2 to 7

$R^5$ and $R^6$ are each hydrogen or $C_{1-6}$ alkyl, or

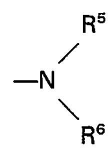

together form a five or six membered ring,

X is —O— or a bond, and

n is 1 or 2.

Preferably, a is 1 or b is 2.

Preferably, X is a bond.

Preferably, n is 1.

Particularly preferred compounds are those wherein $R^3$ is methyl.

Pharmaceutically acceptable salts of compounds of formula (I) include acid addition salts formed with a pharmaceutically acceptable acid such as hydrochloric acid, hydrobromic acid, orthophosphoric acid, sulphuric acid, methanesulphonic acid, toluenesulphonic acid, acetic acid, propionic acid, lactic acid, citric acid, fumaric acid, malic acid, succinic acid, salicylic acid or acetylsalicylic acid.

The present invention also encompasses hydrates of the pharmaceutically acceptable salts of formula (I)

Preferred esters of the compounds of formula (I) are $C_{1-6}$ alkyl esters of the compounds wherein $R^4$ is $-O(CH_2)_aCO_2H$. Particularly preferred esters are methyl and ethyl esters.

Preferred amides of the compounds of formula (I) are those wherein $R^4$ is converted to a group of the formula $-O(CH_2)_aCONR^5R^6$, where $R^5$ and $R^6$ are as defined in formula (I).

When $R^3$ is methyl, the compounds of formula (I) have three asymmetric carbon atoms, marked with asterisks in the formula. These compounds may, therefore, exist in up to eight stereoisomeric forms. The present invention encompasses all stereoisomers of the compounds of formula (I) whether free from other stereoisomers or admixed with other stereoisomers in any proportion and thus includes, for instance, racemic mixtures of enantiomers.

Preferably, all three asymmetric carbon atoms have the R configuration.

The absolute configuration of any compound of formula (i) may be determined by conventional X-ray crystallographic techniques.

In a particularly preferred aspect the present invention provides a compound selected from the group consisting of:
methyl 4-[2-[bis-[β-hydroxy-3-chlorophenethyl]amino]propyl]phenoxyacetate;
ethyl 4-[(R)-2-[bis-[(R)-β-hydroxyphenethyl]amino]propyl]phenoxyacetate;
ethyl 4-[(R)-2-[bis-[(R)-β-hydroxy-3-chlorophenethyl]amino]propyl]phenoxyacetate;
ethyl 4-[(R)-3-[bis-[(R)-β-hydroxyphenethyl]amino]butyl]phenoxyacetate;
4-[(R)-2-[bis[(R)-β-hydroxyphenethyl]amino]propyl]phenoxyethanol;
N-methyl 4-[(R)-2-[bis[(R)-β-hydroxyphenethyl]amino]propyl]phenoxyacetamide;
N-methyl 4-[(R)-2-[bis-[(R)-β-hydroxyphenethyl]amino]propyl]phenoxyethylamine; and
4-[(R)-2-[bis-[(R)-β-hydroxyphenethyl]amino]propoxy]phenoxyacetamide; or a pharmaceutically acceptable salt thereof.

The present invention also provides a process for the preparation of a compound of formula (I) or a pharmaceutically acceptable salt thereof, which comprises reacting a compound of formula (II)

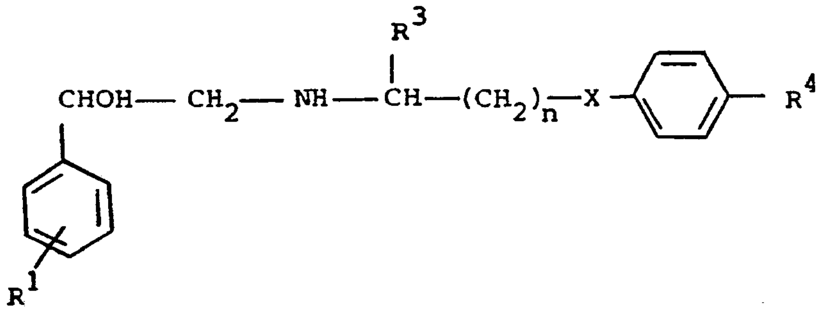

(II)

wherein
$R^1$, $R^3$, $R^4$, X and n are as defined in relation to formula (I);
with a compound of formula (III):

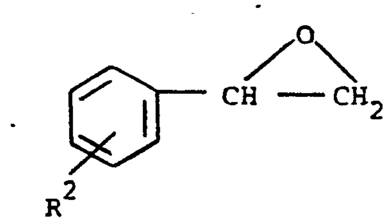

(III)

wherein
$R^2$, is as defined in relation to formula (I); and optionally thereafter converting a compound of formula (I) into a further compound of formula (I) and/or converting a compound of formula (i) to a pharmaceutically acceptable salt thereof.

The reaction is conveniently carried out in dimethyl sulphoxide or a lower alkanol at elevated temperature, optionally in the presence of a catalytic amount of a tertiary amine such as triethylamine.

A compound of formula (I) or a pharmaceutically acceptable salt thereof may also be prepared by reacting the hereinbefore defined compound of formula (II) with a compound of formula (IV):

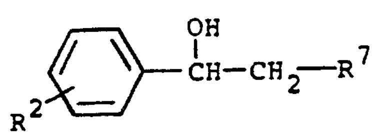

(IV)

wherein
$R^2$ is as defined in relation to formula (I) and $R^7$ is a leaving group; and optionally thereafter converting a compound of formula (I) into a further compound of formula (I) and/or converting a compound of formula (I) thus formed into a pharmaceutically acceptable salt thereof.

Preferably, $R^7$ is a halogen atom, particularly bromine, or a tosyloxy group.

The reaction of a compound of formula (II) with a compound of formula (IV) is conveniently carried out in a solvent, preferbly dimethyl sulphoxide, at elevated temperature, preferably 50°C, for about two or three days.

An alternative process for the preparation of a compound of formula (I) or a pharmaceutically acceptable salt thereof comprises reducing a compound of formula (V):

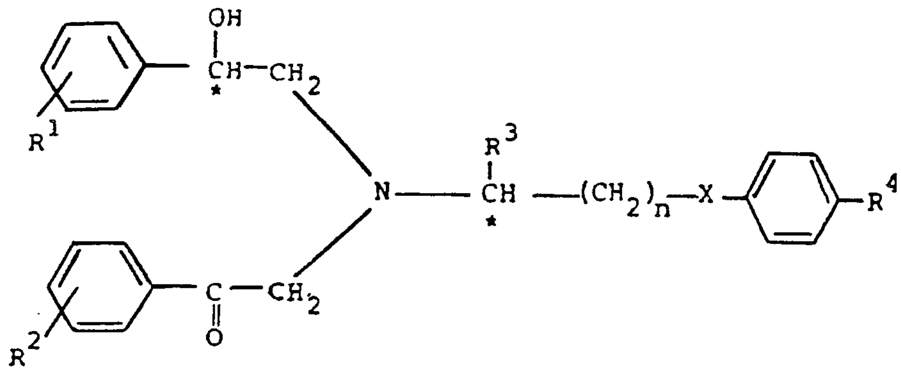

(V)

wherein

$R^1$, $R^2$, $R^3$, $R^4$, X and n are as defined in relation to formula (I); and optionally thereafter converting a compound of formula (I) into a further compound of formula (I) and/or converting a compound of formula (I) thus formed into a pharmaceutically acceptable salt thereof.

Reduction may be effected by conventional chemical methods for example with lithium aluminium hydride, or sodium borohydride.

A compound of formula (I) or a pharmaceutically acceptable salt thereof may also be prepared by alkylating a compound of formula (VI):

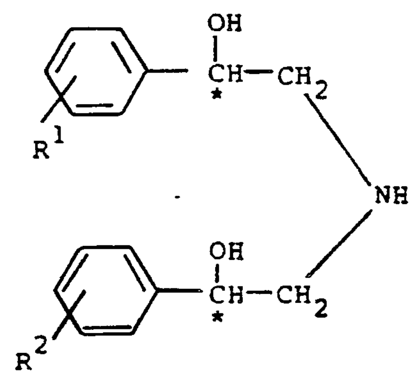

(VI)

wherein

$R^1$ and $R^2$ are as defined in relation to formula (I),

(a) by reaction with a compound of formula (VII)

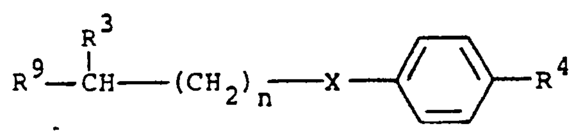

(VII)

wherein

$R^3$, $R^4$, X and n are as defined in relation to formula (I) and $R^9$ is a leaving group; or

(b) by reaction with a compound of formula (VIII)

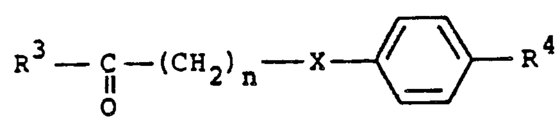

(VIII)

wherein

$R^3$, $R^4$, X and n are as defined in relation to formula (I), in the presence of sodium cyanoborohydride in methanol; and optionally thereafter converting a compound of formula (I) into a further compound of formula (I) and/or converting a compound of formula (I) into a pharmaceutically acceptable salt thereof.

Preferably $R^9$ is a tosyloxy group.

The reaction between the compounds of formula (VI) and the compounds of formula (VII) is conveniently carried out in a solvent, preferably dimethylsulphoxide, at elevated temperature preferably 50°C, for about two or three days.

The compounds of formula (V) may be prepared by reacting a compound of the hereinbefore defined formula (II) with a compound of formula (IX):

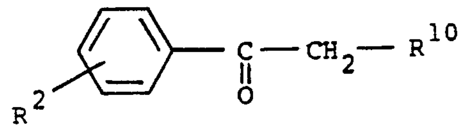

(IX)

wherein

$R^2$ is as defined in relation to formula (I) and $R^{10}$ is a halogen atom, preferably a bromine atom.

The preceding reaction may be carried out in a solvent such as acetonitrile or butanone at an elevated temperature, for example under reflux. An acid acceptor is generally present during the reaction for example a tertiary amine which may be a further mole of the compound of the formula (II).

Compounds of formulae (II), (III), (IV), (VI), (VII), (VIII) and (IX) are either known compounds or can be prepared from known compounds by known processes or processes analogous to known processes.

The salt of compounds of formula (I) may be produced by treating the compound of formula (I) with the appropriate acid.

Compounds of formula (I) and salts thereof, produced by the above process, may be recovered by conventional methods.

Compounds of formula (I) may be separated into diastereoisomeric pairs of enantiomers by, for example, fractional crystallization from a suitable solvent such as methanol or ethyl acetate or a mixture thereof. The pair of enantiomers thus obtained may be separated into individual stereoisomers by conventional means such as by the use of an optically active acid as a resolving agent.

Suitable optically active acids which may be used as resolving agents are described in 'Topics in Stereochemistry' Vol. 6, Wiley Interscience, 1971, Allinger, N. L. and Eliel, W. L. Eds.

Alternatively any enantiomer of a compound of formula (I) may be obtained by stereospecific synthesis using optically pure starting materials of known configuration.

A compound of formula (I) or a pharmaceutically acceptable salt thereof (hereinafter 'the drug') may be administered as the pure drug, however, it is preferred that the drug be administered as a pharmaceutical composition also comprising a pharmaceutically acceptable carrier.

Accordingly, the present invention also provides a pharmaceutical composition comprising a compound of formula (I) or a pharmaceutically acceptable salt thereof with a pharmaceutically acceptable carrier therefor.

As used herein the terms 'pharmaceutical composition' and 'pharmaceutically acceptable' embrace compositions and ingredients for both human and veterinary use; for example the term "pharmaceutically acceptable salts" includes veterinarily acceptable salts.

Usually the compositions of the present invention will be adapted for oral administration although compositions for administration by other routes, such as by injection are also envisaged.

Particularly suitable compositions for oral administration are unit dosage forms such as tablets and capsules. Other fixed unit dosage forms, such as powders presented in sachets, may also be used.

In accordance with conventional pharmaceutical practice the carrier may comprise a diluent, filler, disintegrant, wetting agent, lubricant, colourant, flavourant or the like.

Typical carriers may, therefore, comprise such agents as microcrystalline cellulose, starch, sodium starch glycollate, polyvinylpyrrolidone, polyvinylpolypyrrolidone, magnesium stearate, sodium lauryl sulphate, sucrose and the like.

Most suitably the composition will be provided in unit dose form. Such unit doses will normally comprise 0.1 to 1000 mg of the drug, more usually 0.1 to 500 mg and favourably 0.1 to 250 mg.

The present invention also provides a compound of formula (I) for use as an active therapeutic substance.

The present invention further provides a method for treating hyperglycaemia in a human or non-human animal which method comprises administering an effective, non-toxic amount of a compound of formula (I) or a pharmaceutically acceptable salt thereof to a hyperglycaemic human or non-human animal.

The present invention further provides a method for treating obesity in a human or non-human animal, which method comprises administering an effective, non-toxic amount of a compound of formula (I) or a pharmaceutically acceptable salt thereof to an obese human or non-human animal.

Conveniently, the drug may be administered as a pharmaceutical composition as hereinbefore defined, and this forms a particular aspect of the present invention.

In treating hyperglycaemic or obese humans the drug may be taken in doses, such as those described above, one to six times a day in a manner such that the total daily dose for a 70 kg adult will generally be about 0.1 to 6000 mg, and more usually about 1 to 1500 mg.

In treating hyperglycaemic or obese non-human animals, especially dogs, the drug may be administered by mouth, usually once or twice a day and at about 0.002 mg/kg to 25 mg/kg, for example 0.02 mg/kg to 10 mg/kg.

In a further aspect the present invention also provides a method for improving the weight gain and/or improving the feed utilisation efficiency and/or increasing lean body mass of livestock, which method comprises the administration to livestock of an effective non-toxic amount of a compound of formula (I) or a veterinarily acceptable salt thereof.

Whilst the compounds of formula (I) and the veterinarily acceptable salts thereof may be administered

to any livestock in the abovementioned method, they are particularly suitable for improving the weight gain and/or feed utilisation efficiency and/or lean body mass in poultry, especially turkeys and chickens, cattle, pigs and sheep.

In the preceding method the compounds of formula (I) and veterinarily acceptable salts thereof will normally be administered orally although non-oral modes of administration, for example injection or implantation, are also envisaged. Suitably the compounds are administered in the feed-stuff or drinking water provided for the livestock. Conveniently these are administered in the feed-stuff at from $10^{-3}$ppm — 500 ppm of total daily fed intake, more usually 0.01 ppm to 250 ppm, suitably less than 100 ppm.

The particular formulations used will of course depend upon the mode of administration but will be those used conventionally in the mode of administration chosen.

For administration in feed-stuff the drugs are conveniently formulated as a premix in association with a suitable carrier.

Accordingly, the present invention also provides a veterinarily acceptable premix formulation comprising a compound of (I) or a veterinarily acceptable salt thereof in association with a veterinarily acceptable carrier.

Suitable carriers are inert conventional agents such as powdered starch. Other conventional feed-stuff premix carriers may also be employed.

No toxicological effects are indicated when a compound of formula (I) or a pharmaceutically acceptable salt thereof is administered in any of the above mentioned dosage ranges.

The invention will now be illustrated with reference to the following Examples, in which the substituents are as defined in the following Table.

| Experiment No. | $R^1$ | $R^2$ | $R^3$ | $R^4$ | X | n | Salt |
|---|---|---|---|---|---|---|---|
| 1 | 3-Cl | 3-Cl | $CH_3$ | $OCH_2CO_2Me$ | - | 1 | HCl |
| 2 | H | H | $CH_3$ | $OCH_2CO_2Et$ | - | 1 | HCl |
| 3 | 3-Cl | 3-Cl | $CH_3$ | $OCH_2CO_2Et$ | - | 1 | HCl |
| 4 | H | H | $CH_3$ | $OCH_2CO_2Et$ | - | 2 | HCl |
| 5 | H | H | $CH_3$ | $OCH_2CH_2OH$ | - | 1 | HCl |
| 6 | H | H | $CH_3$ | $OCH_2CONHMe$ | - | 1 | HCl |
| 7 | H | H | $CH_3$ | $OCH_2CH_2NHMe$ | - | 1 | HCl |
| 8 | H | H | $CH_3$ | $OCH_2CONH_2$ | O | 1 | HCl |

## Example 1

Methyl 4-[2-[bis-[β-hydroxy-3-chlorophenethyl]amino]propyl]phenoxyacetate hydrochloride monohydrate

To a solution of (RR,SS)-N-[2-(4-carbomethoxymethoxyphenyl)-1-methylethyl)-2-hydroxy-2-(3-chlorophenyl)ethanamine (8.5 g) in methanol (100 ml) containing triethylamine (3 ml) was added 3-chlorostyrene oxide (2.8 g). The solution was boiled under reflux with stirring for 72 hours, cooled and the solvent removed under reduced pressure. The residual oil was chromatographed on silica-gel in methanol-dichloromethane mixtures (2—10%). Treatment of the product thus obtained with ethereal hydrogen chloride gave the title compound m.p. 128—30°C.

$^1$H nmr δ(DMSO-$d_6$)

1.15 (3H, complex); 2.55—4.30 (9H, complex); 3.70 (3H, s); 4.75 (2H, s); 5.2—5.6 (2H, complex); 6.3—6.9 (2H, broad d, exchanges with $D_2O$); 6.85 (2H, d); 7.15—7.70 (10H, complex); 8.80—9.65 (1H, broad d, exchanges with $D_2O$).

## Example 2

Ethyl 4-[(R)-2-[bis-[(R)-β-hydroxyphenethyl]amino]propyl]phenoxyacetate hydrochloride

A mixture of (R)-styrene oxide (7.72 g) and methyl 4-[(R)-2-aminopropyl]phenoxyacetate (7.17 g) in ethanol (100 ml) was heated under reflux for 72h. The solvent was evaporated and the residual oil was chromatographed on silica. Elution with chloroform gave an oil which, after treatment with ethereal

hydrogen chloride, gave ethyl 4-[(R)-2-[bis-[(R)-β-hydroxyphenethyl]amino]propyl]phenoxyacetate hydrochloride, m.p. 72—75° (benzene-ether), $[\alpha]_D^{25}$ (MeOH), −102° (c=0.8).

$^1$H nmr δ(DMSO-$d_6$)

1.22 (6H, m); 2.83—2.95 (1H, m); 3.20—3.60 (5H, m); 4.05 (1H, m); 4.18 (2H, q); 4.76 (2H, s); 5.30 (2H, m); 6.45 (2H, m, exchanges with $D_2O$); 6.90 (2H, d); 7.25—7.55 (12H,m); 8.80 (1H, broad, exchanges with $D_2O$).

Ethyl 4-[(R)-2-[bis-[(R)-β-hydroxy-3-chlorophenethyl]amino]propyl]phenoxyacetate hydrochloride

Ethyl 4-[(R)-2-[bis-[(R)-β-hydroxy-3-chlorophenethyl]amino]propyl]phenoxyacetate hydrochloride. m.p. 63-67°, was prepared from (R)-3-chlorostyrene oxide and methyl 4-[(R)-2-aminopropyl]phenoxyacetate by an analogous procedure to that described in Example 2.

$^1$H nmr δ(DMSO-$d_6$)

1.18—1.24 (6H, m); 2.88—3.00 (1H, m); 3.12—3.60 (5H, m); 3.92—4.08 (1H, m); 4.13—4.21 (2H, q); 4.74 (2H, s); 5.17—5.28 (2H, m); 6.54—6.62 (2H, m, exchanges with $D_2O$); 6.91 (2H, d); 7.30 (2H, d); 7.35—7.62 (8H, m); 8.25 (1H, broad, exchanges with $D_2O$).

## Example 4

Ethyl 4-[(R)-2-[bis-[(R)-β-hydroxyphenethyl]amino]butyl]phenoxyacetate hydrochloride

Ethyl 4-[(R)-3-[bis-[(R)-β-hydroxyphenethyl]amino]butyl]phenoxyacetate hydrochloride, isolated as a hard foam, was prepared from (R)-styrene oxide and methyl 4-[(R)-3-aminobutyl]phenoxyacetate by an analogous procedure to that described in Example 2.

$^1$N nmr δ(DMSO-$d_6$)

1.18 (3H, t); 1.34 (3H, d); 1.80—1.98 (1H, m); 2.03—2.21 (1H, m); 2.58—2.68 (2H, m); 3.18—3.58 (4H, m); 3.80—3.93 (1H, m); 4.16 (2H, q); 4.75 (2H, s); 5.13—5.31 (2H, m); 6.58 (1H, m, exchanges with $D_2O$); 6.71 (1H, m, exchanges with $D_2O$); 6.87 (2H, d); 7.20 (2H, d); 7.23—7.56 (10H,m); 8.37 (1H, broad, exchanges with $D_2O$).

## Example 5

4-[(R)-2-[Bis-[β-hydroxyphenethyl]amino]propyl]phenoxy ethanol hydrochloride

Sodium borohydride (1 g) was added in portions to a stirred solution of ethyl 4-[(R)-2-[bis-[(R)-β-hydroxyphenethyl]amino]propyl]phenoxyacetate in methanol (50 ml). The solution was left to stand at ambient temperature for 20h, evaporated and the residue partitioned between ethyl acetate and water. The organic phase gave an oil which was purified by column chromatography on silica. Elution with methanolchloroform (1:99) gave an oil which, after treatment with ethereal hydrogen chloride, gave 4-[(R)-2-[bis-[(R)-β-hydroxyphenethyl]amino]propyl]phenoxyethanol hydrochloride, m.p. 72—77° (ether-acetone), $[\alpha]_D^{25}$ (MeOH), −107° (c=0.8).

$^1$N nmr δ(DMSO-$d_6$)

1.20 (3H, d); 2.80—2.95 (1H, m); 3.15—3.60 (5H, m); 3.70 (2H, t); 3.95—4.10 (2H, t plus 1H, m); 4.85 (1H, broad, exchanges with $D_2O$); 5.27 (2H, m); 6.40—6.50 (2H, m, exchanges with $D_2O$); 6.90 (2H, d); 7.25—7.60 (12H, m); 8.75 (1H, s, exchanges with $D_2O$).

## Example 6

N-Methyl 4-[(R)-2-[bis-[(R)-β-hydroxyphenethyl]amino]propyl]phenoxyacetamide hydrochloride

Methylamine (25 ml of a 33% solution in ethanol) was added dropwise to a stirred solution of ethyl 4-[(R)-2-[bis-[(R)-β-hydroxyphenethyl]amino]propyl]phenoxyacetate (4 g) in methanol (80 ml) at reflux. The solution was allowed to stand at ambient temperature for 20 h and then evaporated to give an oil which was purified by column chromatography on silica. Elution with methanol-chloroform (1:99) gave a foam which, after treatment with ethereal hydrogen chloride, gave N-methyl 4-[(R)-2-[bis-[(R)-β-hydroxyphenethyl]amino]propyl]phenoxyacetamide hydrochloride, m.p. 92—97°, (THF-ether), $[\alpha]_D^{25}$ (MeOH) −100° (c=0.9).

$^1$H nmr δ(DMSO-$d_6$)

1.20 (3H, d); 2.65 (3H, d); 2.80—2.95 (1H, m); 3.20—3.60 (5H, m); 3.95—4.10 (1H, m); 4.44 (2H, s); 5.30 (2H, m); 6.40—6.50 (2H, m, exchanges with $D_2O$); 6.93 (2H, d); 7.30—7.55 (12H, m); 8.05 (1H, m, exchanges with $D_2O$); 8.95 (1H, s, exchanges with $D_2O$).

## Example 7

N-Methyl 4-[(R)-2-[bis-[(R)-β-hydroxyphenethyl]amino]propyl]phenoxy ethylamine dihydrochloride

Borane methyl sulphide (0.58 g) in dry tetrahydrofuran (5 ml) was added, dropwise, under nitrogen to a stirred solution of N-methyl 4-[(R)-2-[bis-[(R)-β-hydroxyphenethyl]amino]propyl]phenoxyacetamide (1.78 g) in dry tetrahydrofuran (40 ml). The solution was stirred and heated under reflux for 18h, cooled, treated with methanol (2 ml) and heated for a further hour. Hydrogen chloride was passed into the resulting solution which was evaporated to a foam. Trituration with ethyl acetate gave N-methyl [4-[(R)-2-[bis-[(R)-β-hydroxyphenethyl]amino]propyl]phenoxyethylamine dihydrochloride, m.p. 145—150°, $[\alpha]_D^{25}$ (MeOH) −87° (c=0.9).

$^1$H nmr δ(DMSO-$d_6$)

1.20 (3H, d); 2.60 (3H, m); 2.80—2.95 (1H, m); 3.20—3.65 (7H, m); 3.95—4.10 (1H, m); 4.25 (2H, m); 5.30 (2H,

m); 6.25—6.70 (2H, broad, exchanges with $D_2O$); 6.95 (2H, d); 7.25—7.55 (12H, m); 8.95 (1H, s, exchanges with $D_2O$); 9.27 (2H, s, exchanges with $D_2O$).

Example 8

4-[(R)-2-[bis-[(R)-β-hydroxyphenethyl]amino]propoxy]phenoxyacetamide hydrochloride

4-[(R)-2-[bis-[(R)-β-hydroxyphenethyl]amino]propoxy]phenoxyacetamide hydrochloride, isolated as a hard foam, was prepared from (R)-styrene oxide and 4-[(R)-2-aminopropoxy]phenoxyacetamide by an analogous procedure to that described in Example 2.

$^1$H nmr δ(DMSO-$d_6$)

0.98 (3H,d); 2.55—2.75 (4H, m); 3.15—3.30 (1H, m); 4.35 (2H, s); 4.55—4.65 (2H, m); 5.10 (2H, s, exchanges with $D_2O$); 6.80—6.95 (4H, q); 7.2—7.50 (13H, m, 3H exchangeable with $D_2O$).

Demonstration of effectiveness of compounds

a) Anti-hyperglycaemic activity

Female CFLP mice, weighing approximately 25 g, were fasted for 24 hours prior to the study. The compound under study was administered orally as an aqueous solution to each of 6 mice. 30 minutes later a blood sample (10 μl) was obtained from the tail for the analysis of blood glucose. Immediately after taking this blood sample, glucose (1 g/kg body weight) was administered subcutaneously to each mouse. 6 mice were given water as a control. Blood samples were then obtained from each mouse at 30 minute intervals for 120 minutes.

Compounds that produce a significant ($P<0.05$) reduction of blood glucose, compared with control mice given water, at any time interval, are considered active. The area under the blood glucose curve over the 2 hour period after the administration of the glucose was calculated for the compounds and compared with the value for control animals. The results for the compounds are as follows:

| Compound of Example No. | Dose (μmol/kg) | % Reduction in area under Blood Glucose Curve |
|---|---|---|
| 1 | 5 | 55 |
| 2 | 2.5 | 52 |
| 4 | 12.5 | 42 |
| 5 | 2.5 | 54 |
| 6 | 2.5 | 52 |
| 7 | 2.5 | 50 |

b) Effect on Energy Expenditure of Mice

The effect of the compound on the energy expenditure of mice was demonstrated by means of the following procedure:

Female CDI mice, each weighing approximately 24 g were given food and water *ad lib* before and during the experiment. The compound was dissolved in water and the solution was administered orally to each of 6 mice. A further 9 mice were dosed orally with water. The mice were placed in groups of three in boxes through which air was drawn and the oxygen content of the air leaving the boxes was measured. The energy expenditure of the mice was calculated for 3 hours and for 21 hours after dosing from the volume of air leaving the boxes and its oxygen content, following the principles described by J.B. de V. Weir, *J. Physiol.* (London), *109*, 1—9 (1949). The results are expressed as a percentage of the rate of energy expenditure of the mice dosed with water. The results for the compounds are as follows:

| Compound of Example No. | Dose mg/kg p.o. | Mean Energy Expenditure (0—3h) | (9—21h) |
|---|---|---|---|
| 1 | 29.3 | 151 | 128 |

c) Effect on Energy Expenditure of Rats

The effect of the compounds on the energy expenditure of rats was demonstrated by means of the following procedure:

Male Sprague-Dawley rats each weighing between 170—200 g were deprived of food for 16 hours before, and during the experiment. Water was provided *ab lib* at all times. The compounds were administered orally in water to 3 or 4 rats. A further 4 rats were dosed orally with water. The rats were placed in boxes through which air was drawn and the oxygen content of the air leaving the boxes was measured. The energy expenditure of the rats was calculated for 3 hours and for 21 hours after dosing from

the volume of air leaving the boxes and its oxygen content, following the pinciples described by J. B. de V. Weir, *J. Physiol.* (London) 109, 1—9 (1949). The results are expressed as a percentage of the rate of energy expenditure of the rats dosed with water. Results are given in Table 1.

| Compound of Example No. | Dose mg/kg p.o. | Mean Energy Expenditure (0—3h) | (9—21h) |
|---|---|---|---|
| 1 | 0.57 | 132 | 110 |
| 2 | 10.3 | 146 | 143 |
| 3 | 5.8 | 144 | 132 |
| 4 | 26.4 | 111 | 99 |
| 5 | 9.4 | 159 | 152 |
| 6 | 10.0 | 154 | 147 |
| 7 | 10.4 | 145 | 146 |
| 8 | 4.73 | 113 | 103 |

d) Anti-Obesity Activity

Female BALB/c mice were trained to feed on powdered oxoid for 1 week. They were then housed in threes and the compound under study was added to the diet for 3 days. Food was removed at 09.00h and the mice were killed at 11.00h. The parmetrial fat pads were weighed as pairs. The result is a mean of 9 values.

The compound of Example Number 3 gave a 38% reduction in weight of perigenital adipose tissue in treated animals compared with controls when given at a dose of 5.8 mg/kg of diet.

**Claims**

1. A compound of formula (I):

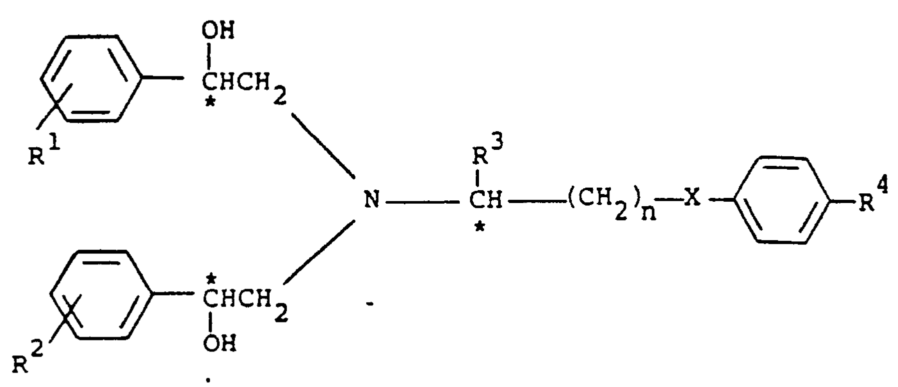

(I)

or a pharmaceutically acceptable salt thereof, in which

each of $R^1$ and $R^2$, which may be the same or different, is hydrogen, halogen, or trifluoromethyl,

$R^3$ is hydrogen or methyl,

$R^4$ is $-O(CH_2)_aCO_2H$, $-O(CH_2)_bOH$ or

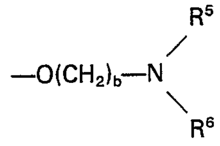

or an ester or amide derivative thereof, in which

a is an integer from 1 to 6

b is an integer from 2 to 7

$R^5$ and $R^6$ are each hydrogen or $C_{1-6}$ alkyl, or

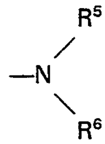

together form a five or six membered ring,

X is —O— or a bond, and

n is 1 or 2.

2. A compound as claimed in claim 1, wherein a is 1 or b is 2.

3. A compound as claimed in claim 1 or claim 2, wherein X is a bond.

4. A compound as claimed in any one of claims 1 to 3, wherein n is 1.

5. A compound as claimed in any one of claims 1 to 4, wherein $R^3$ is methyl.

6. A compound as claimed in claim 1, selected from the group consisting of:

methyl 4-[2-[bis-[β-hydroxy-3-chlorophenethyl]amino]propyl]phenoxyacetate;

ethyl 4-[(R)-2-[bis-[(R)-β-hydroxyphenethyl]amino]propyl]phenoxyacetate;

ethyl 4-[(R)-2-[bis-[(R)-β-hydroxy-3-chlorophenethyl]amino]propyl]phenoxyacetate;

ethyl 4-[(R)-3-[bis-[(R)-β-hydroxyphenethyl]amino]propyl]phenoxyacetate;

4-[(R)-2-[bis[(R)-β-hydroxyphenethyl]amino]propyl]phenoxyethanol;

N-methyl 4-[(R)-2-[bis[(R)-β-hydroxyphenethyl]amino]propyl]phenoxyacetamide;

N-methyl 4-[(R)-2-[bis[(R)-β-hydroxyphenethyl]amino]propyl]phenoxyethylamine; and

4-[(R)-2-[bis[(R)-β-hydroxyphenethyl]amino]propoxy]phenoxyacetamide; or a pharmaceutically acceptable salt thereof.

7. A process for the preparation of a compound formula (I) or a pharmaceutically acceptable salt thereof, which comprises reacting a compound of formula (II);

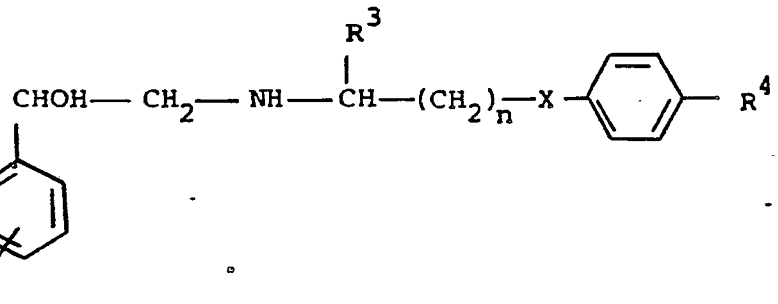

(II)

wherein

$R^1$, $R^3$, $R^4$, X and n are as defined in relation to formula (I);

with a compound of formula (III):

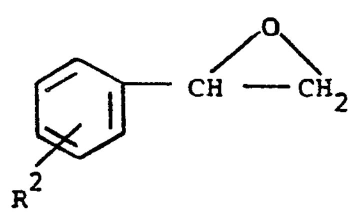

(III)

wherein

$R^2$, is as defined in relation to formula (I); and optionally thereafter converting a compound of formula (I) into a further compound of formula (I) and/or converting a compound of formula (I) to a pharmaceutically acceptable salt thereof.

8. A pharmaceutical composition comprising a compound of formula (I), as defined in claim 1, or a pharmaceutically acceptable salt thereof with a pharmaceutically acceptable carrier therefor.

9. A compound of formula (I), as defined in claim 1, or a pharmaceutically acceptable salt thereof, for use as an active therapeutic substance.

10. A method for improving the weight gain and/or improving the feed utilisation efficiency and/or increasing lean body mass of livestock, which method comprises the administration to livestock of an effective non-toxic amount of a compound of formula (I) as defined in claim 1 or a veterinarily acceptable salt thereof.

**Revendications**

1. Composé de formule (I):

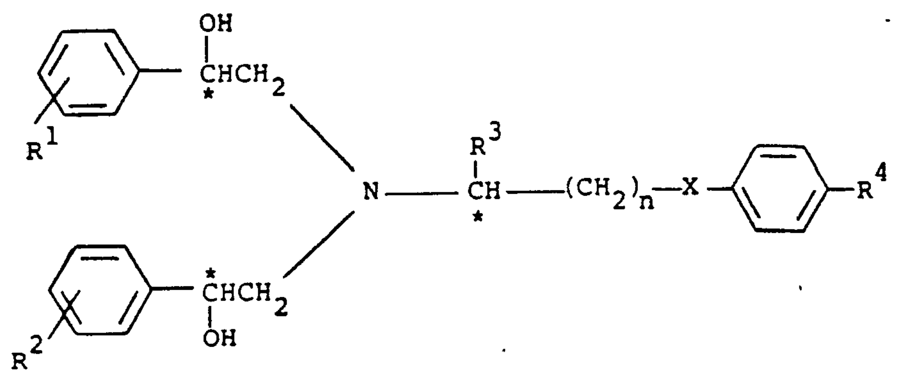

(I)

ou un sel de celui-ci acceptable du point de vue pharmaceutique, dans laquelle:

chacun des $R^1$ et $R^2$, qui peuvent être identiques ou différents, est un atome d'hydrogène, d'halogène ou un groupe trifluorométhyle,

$R^3$ est un atome d'hydrogène ou un groupe méthyle,

$R^4$ est un radical $—O(CH_2)_aCO_2H$, $—O(CH_2)_bOH$ ou

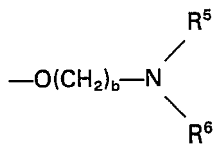

ou un dérivé ester ou amide de ceux-ci, dans lesquels

a est un entier de 1 à 6,

b est un entier de 2 à 7,

$R^5$ et $R^6$ sont chacun un atome d'hydrogène ou un groupe alcoyle en $C_{1-6}$, ou bien

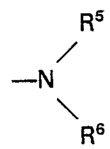

forment ensemble un novau à cinq ou six chaînons,

X est —O— ou une liaison,

et n est 1 ou 2.

2. Composé suivant la revendication 1, caractérisé en ce que a est l ou b est 2.

3. Composé suivant la revendication 1 ou la revendication 2, caractérisé en ce que X est une liaison.

4. Composé suivant l'une quelconque des revendications 1 à 3, caractérisé en ce que n est 1.

5. Composé suivant l'une quelconque des revendications 1 à 4, caractérisé en ce que $R^3$ est un groupe méthyle.

6. Composé suivant la revendication 1, caractérisé en ce qu'il est choisi dans le groupe comprenant:
le 4-[2-[bis-[β-hydroxy-3-chlorophénéthyl]amino]propyl]phénoxyacétate de méthyle;
le 4-[(R)-2-[bis-[(R)-β-hydroxyphénéthyl]amino]propyl]phénoxyacétate d'éthyle;
le 4-[(R)-2-[bis-[(R)-β-hydroxy-3-chlorophénéthyl]amino]propyl]phénoxyacétate d'éthyle;
le 4-[(R)-3-[bis-[(R)-β-hydroxyphénéthyl]amino]butyl]phénoxyacétate d'éthyle;
le 4-[(R)-2-[bis[(R)-β-hydroxyphénéthyl]amino]propyl]phénoxyéthanol;

le N-méthyl 4-[(R)-2-[bis[(R)-β-hydroxyphénéthyl]amino]propyl]phénoxyacétamide;
la N-méthyl 4-[(R)-2-[bis[(R)-β-hydroxyphénéthyl]amino]propyl]phénoxyéthylamine; et le
4-[(R)-2-[bis[(R)-β-hydroxyphénéthyl]amino]propoxy]phénoxyacétamide; ou un sel de ceux-ci acceptable du point de vue pharmaceutique.

7. Procédé pour la préparation d'un composé de formule (I) ou d'un sel de celui-ci acceptable du point de vue pharmaceutique, caractérisé en ce qu'il comprend la réaction d'un composé de formule (II):

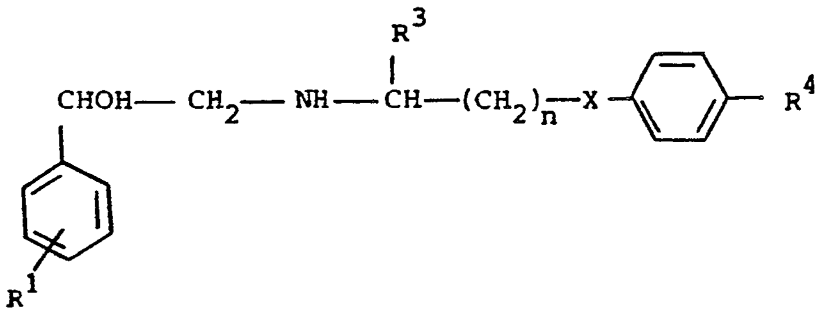

(II)

dans laquelle $R^1$, $R^3$, $R^4$, X et n sont tels que définis à propos de la formule (I); avec un composé de formule (III):

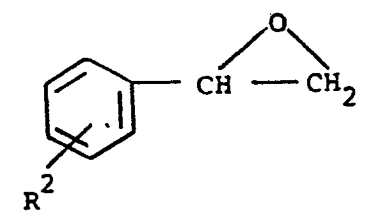

(III)

dans laquelle $R^2$ est tel que défini à propos de la formule (I); et ensuite éventuellement la conversion d'un composé de formule (I) en un autre composé de formule (I) et/ou la conversion d'un composé de formule (I) en un sel se celui-ci acceptable pharmaceutiquement.

8. Composition pharmaceutique comprenant un composé de formule (I) suivant la revendication 1 ou un sel de celui-ci acceptable du point de vue pharmaceutique avec un support pour celui-ci acceptable du point de vue pharmaceutique.

9. Composé de formule (I) suivant la revendication 1 ou un sel de celui-ci acceptable du point de vue pharmaceutique utile comme substance thérapeutique active.

10. Méthode pour améliorer la prise de poids et/ou améliorer l'efficacité de l'utilisation des aliments et/ou augmenter la masse corporelle maigre des animaux sur pied, caractérisé en ce qu'elle comprend l'administration à ceux-ci d'une quantité non-toxique efficace d'un composé de formule (I) suivant la revendication 1 ou d'un sel de celui-ci acceptable du point de vue vétérinaire.

**Patentansprüche**

1. Eine Verbindung der Formel (I):

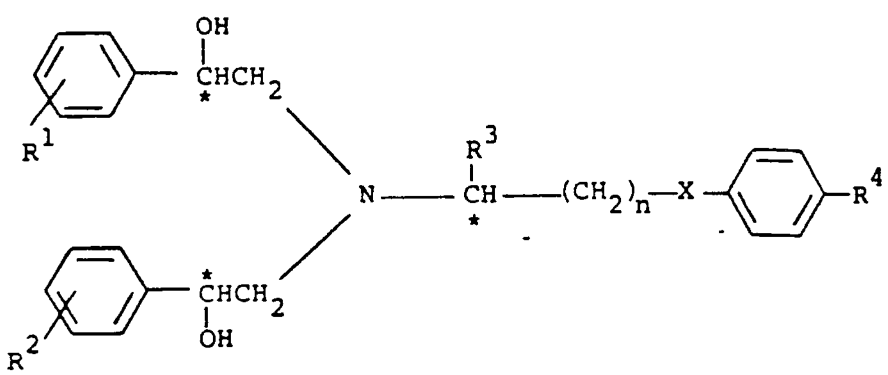

(I)

oder ein pharmazeutisch verträgliches Salz davon, in welcher jede der beiden Gruppen $R^1$ und $R^2$, welche gleich oder verschieden sein können, Wasserstoff, Halogen oder Trifluormethyl bedeutet,

$R^3$ Wasserstoff oder Methyl ist,

$R^4$ eine Gruppe $—O(CH_2)_aCO_2H$, $—O(CH_2)_bOH$ oder

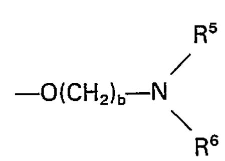

oder ein Ester oder Amidderivat davon ist, wobei a eine ganze Zahl von 1 bis 6 ist
b eine ganze Zahl von 2 bis 7 ist,
$R^5$ und $R^6$ jeweils Wasserstoff oder $C_{1-6}$-Alkyl bedeuten, oder

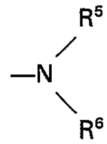

zusammen einen 5- oder 6-gliedrigen Ring bilden,
X —O— oder eine Bindung darstellt, und n 1 oder 2 ist.

2. Eine Verbindung wie in Anspruch 1 beansprucht, in welcher a den Wert 1 oder b den Wert 2 hat.

3. Eine Verbindung wie in Anspruch 1 oder 2 beansprucht, in welcher X eine Bindung darstellt.

4. Eine Verbindung wie in einem der Ansprüche 1 bis 3 beansprucht, in welcher n den Wert 1 hat.

5. Eine Verbindung wie in einem der Ansprüche 1 bis 4 beansprucht, in welcher $R^3$ Methyl ist.

6. Eine Verbindung wie in Anspruch 1 beansprucht, ausgewählt aus der Gruppe bestehend aus:
Methyl 4-[2-[bis-[β-hydroxy-3-chlorphenäthyl]amino]propyl]phenoxyacetat;
Äthyl 4-[(R)-2-[bis-[(R)-β-hydroxyphenäthyl]amino]propyl]phenoxyacetat;
Äthyl 4-[(R)-2-[bis-[(R)-β-hydroxy-3-chlorphenäthyl]amino]propyl]phenoxyacetat;
Äthyl 4-[(R)-3-[bis-[(R)-β-hydroxyphenäthyl]amino]butyl]phenoxyacetat;
4-[(R)-2-[bis[(R)-β-hydroxyphenäthyl]amino]propyl]phenoxyäthanol;
N-Methyl 4-[(R)-2-[bis[(R)-β-hydroxyphenäthyl]amino]propyl]phénoxyacetamid;
N-Methyl 4-[(R)-2-[bis[(R)-β-hydroxyphenäthyl]amino]propyl]phenoxyäthylamin; und
4-[(R)-2-[bis[(R)-β-hydroxyphenäthyl]amino]propoxy]phenoxyacetamid; oder ein pharmazeutisch verträgliches Salz davon.

7. Ein Verfahren zur Herstellung einer Verbindung der Formel(I) oder eines pharmazeutisch verträglichen Salzes davon, welches die Umsetzung einer Verbndung der Formel (II):

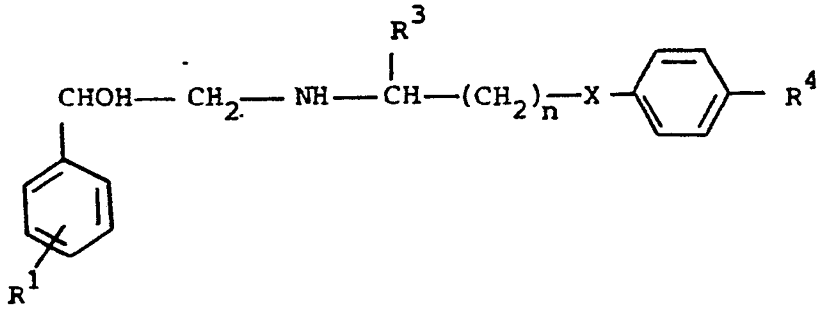

(II)

in welcher
$R^1$, $R^3$, $R^4$, X und n wie in bezug auf Formel(I) definiert sind;
mit einer Verbindung der Formel(III):

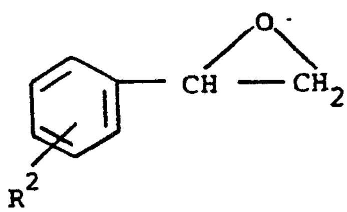

(III)

in welcher $R^2$ wie in bezug auf Formel (I) definiert ist; und gegebenenfalls anschließend die Umwandlung einer Verbindung der Formel(I) in eine weitere Verbindung der Formel(I) und/oder die Umwandlung einer Verbindung der Formel(I) in ein pharmazeutisch verträgliches Salz davon, umfaßt.

8. Ein pharmazeutische Zusammensetzung umfassend eine Verbindung der Formel(I), wie in Anspruch 1 definiert, oder ein pharmazeutisch verträgliches Salz davon mit einem pharmazeutisch verträglichen Träger dafür.

9. Eine Verbindung der Formel(I), wie in Anspruch 1 definiert, oder ein pharmazeutisch verträgliches Salz davon, zur Verwendung als aktive therapeutische Substanz.

10. Ein Verfahren zur Verbesserung der Gewichtszunahme und/oder zur Verbesserung der Nahrungsverwertung und/oder zur Erhöhung des mageren Fleischanteils bei Vieh, welches Verfahren die Verabreichung einer wirksamen nicht-toxischen Menge einer Verbindung der Formel(I), wie in Anspruch 1 beansprucht, oder eines veterinärmedizinisch verträglichen Salzes davon an Vieh umfaßt.